Europäisches Patentamt

European Patent Office

Office européen des brevets

(19)

(11) Publication number:

0 354 580
A2

# EUROPEAN PATENT APPLICATION

(21) Application number: 89114857.9

(22) Date of filing: 10.08.89

(51) Int. Cl.⁴ **C07H 21/04** , **C07K 13/00** , **C12N 5/00** , //C12N15/12, **C12Q1/68**

(30) Priority: 11.08.88 JP 200758/88

(43) Date of publication of application:
**14.02.90 Bulletin 90/07**

(84) Designated Contracting States:
**DE FR GB**

(71) Applicant: OLYMPUS OPTICAL CO., LTD.
43-2, 2-chome, Hatagaya Shibuya-ku
Tokyo 151(JP)

(72) Inventor: **Kano, Kyoichi**
1-4-33-702, Takanawa Minato-ku
Tokyo(JP)
Inventor: **Takiguchi, Masafumi**
6-3-4, Minamisenju Arakawa-ku
Tokyo(JP)

(74) Representative: **Kuhnen, Wacker & Partner**
Schneggstrasse 3-5 Postfach 1553
D-8050 Freising(DE)

(54) HLA class I DNA, DNA probes and transformed cells.

(57) Two HLA class I DNAS, which have a exon represented by the base sequence specified in claim 1 and a exon represented by the base sequence specified in claim 2, respectively, encode for HLA-B51 antigen and HLA-Bw52 antigen, respectively. DNA probes for use in DNA typing can be obtained from both DNAs, by labeling a marker substance to a part of the DNAs. Transformed cells, which are useful as immunogens, can be obtained by introducing these DNAS into eucaryotic cells.

EXON    1   2       3       4   5   6   7

5'-TERMINAL   L   α1      α2      α3  TM  C1  C2  3'-UT

# F I G. 1

## HLA class I DNA, DNA probes and transformed cells

This invention relates to HLA (human leukocyte antigen) class I DNA, DNA probes and transformed cells, particularly to DNA coding for allotypes of HLA-B antigens, DNA probes utilized for separating and identifying the HLA class I genes themselves of a patient through DNA typing, and transformed cells obtained by introducing the DNA of the present invention into eucaryotic cells of animals other than human beings and which express allotypes of HLA-B antigens on their cellular surfaces.

HLA antigens play a significant part in transplantation of human organs through transplantation immunity, and function as antigens which determine whether or not transplanted organs or tissues are acceptable. Among HLA antigens, class I antigens are concerned in antigen recognition through cytotoxic T lymphocytes in cell-mediated immunity, and also manifestly have significant correlations with diseases such as psoriasis (cw6), pemphigus(A10), herpes labialis (A29), Behcet's syndrome (B57) (Ozawa, A., Ohkido, M., Tsuji, K., J. Am. Acad. Dermatol, 4, 205, 1981).

HLA antigens are called major histocompatibility antigens (MHA) for profoundly involved in rejection and transplantation immunity. Gene groups coding for these antigens are accordingly called major histocompatibility complex (MHC).

HLA are human MHA, and HLA class I antigens are membrane protein on cell membranes and controlled by three gene loci comprising HLA-A, HLA-B, and HLA-C. A class I antigen comprises glycoprotein (alpha-chain) of a 44kd molecule weight associated with protein ($\beta_2$-microglobulin) of a 12kd molecular weight through non-covalent bond. The alpha-chains are composed of three domains such as $\alpha_1$, $\alpha_2$, and $\alpha_3$ at extracellular N-terminus site, followed by transmembrane region which is embedded in cell membrane and intracytoplasmic region which locate in the cell. The $\beta_2$-microglobulin is composed of one domain and combined with the alpha-chain.

HLA gene region is present on short-arm of human sixth chromosome over 2.5 centimorgan (cM) having from a centromere site class II region (1.0cM), class III region (DR-B distance = 0.7cM), and class I region (0.8cM) in this order as shown in Fig. 4 (Jordan, B.R. et al. Immunol. Rev., 84,73,1985).

Methods for detecting HLA class I antigens include serological method and HLA-DNA typing method through recombinant DNA technique.

A typical serological method is complement-dependent cytotoxicity test (Terasaki, P,I. and McClelland, J.D. Nature (London), 204, pp 998-1000, 1964). This method introduces alloantiserum into a sample and types HLA by means of lymphocytetoxicity utilizing rabbit's serum as complement. In this method, however, the antiserum must be alloserum of a grand multipara. In addition, since the antiserum shows cross reaction, antiserum which specifically react with HLA class I allotype cannot be easily obtained. Monoclonal antibodies which replace the alloserum are thus expected to be produced. Approximately 150 kinds of monoclonal antibodies have been introduced in The International Histocompatibility Workshop in 1984 (Fauchet, R., Bonder, J.G., Kennedy, L.J. et al, : HLA-A, B, C monoclonal antibodies. Histocompatibility Testing 1984 (Albert, E.D., Baur, M.P., Mayr, W.R. ed), Springer-Verlag, Berlin, Heidelberg, New York, Tokyo: 211, 1984). Anti-HLA monoclonal antibodies are generally produced by a method which fuses mouse splenocyto derived from a mouse immuned by human lymphocytes and mouse myeloma cells (Oi, V.T., Herzenberg, L.A.: Immunoglobulin-producing hybrid cell lines. Selected methods in Immunology. 351, 1981). However, among monoclonal antibodies produced up to date, for example, antibodies suitable for HLA typing such as antibodies against allotypes of HLA-B antigens include monoclonal antibodies which specifically combine with Bw4, Bw6, B7, B8, B13, and B27 only. Thus, monoclonal antibodies having specificity with other allotypes are expected to be invented. Recently an improved method has been invented for producing anti-HLA monoclonal antibodies. In conventional method for producing antibodies utilizing human T lymphocyte-dominant peripheral blood lymphocytes as immunogens, since many antigens derived from human beings are inconveniently presented, monoclonal antibodies which are reactive with many epitopes other than objective HLA allotype antigens are obtained. The improved method is circumvented in this respect over the conventional method by introducing gene clone of human HLA antigens into eucaryocytes derived from immuned animal to obtain transformed cells, administration said transformed cells to said immuned animals to obtain splenocyto which efficiently produce monoclonal antibodies against human HLA antigens expressed on the transfected cells (Monoclonal antibodies to HLA-DP-transfected mouse L cell: Proc. Natl. Acad. Sci., USA, 83, pp 3417-3421, 1986).

For HLA class I antigens for which antiserum or monoclonal antibodies having specificity with the antigen allotypes are not available, DNA typing is an effective method to detect the antigens. For example, psoriasis is known to highly correlate with Cw6 and Cw7 of HLA class I antigen (Ozawa et al.: Some recent advances in HLA and skin diseases, J.Am.Acad.Dermatol.4, 205, 1981). Ozawa et al. conducted DNA typing on 21 patients of psoriasis having Cw6 and Cw7 and 16 healthy persons by utilizing Pvu II fragment of C antigen gene clone p 42 (Sodoyer, R. et al., complete nucleotide sequence of a gene encoding a functional human class I histocompatibility antigen (HLA-Cw3), EMBO J., 3:879, 1984) as probes (Ozawa, A. et al: DNA typing in psoriasis Vulgaris, In: Proceeding of the 3rd Asia Oceania Histocompatibility Workshop and Conference, in press). Consequently, when 1.7 kb fragment of Pvu II corresponding to signal sequence, $\alpha_1$ domain and a part of $\alpha_2$ domain is utilized as probes, a specific band was detected for the patients.

As noted above, the conventional method is restricted in HLA antigen allotype analysis and DNA typing application because the number of HLA-DNA which are separated and whose base sequences are determined is not so large. Moreover, the number of successful immunogens is small for producing anti-HLA monoclonal antibodies.

A primary object of the present invention is to produce HLA class I DNA having exon of the following base sequence;

```
ATGCGGGTCACGGCGCCCCGAACCGTCCTCCTGCTGCTCT
GGGGGGCAGTGGCCCTGACCGAGACCTGGGCCGCTCCCAC
TCCATGAGGTATTTCTACACCGCCATGTCCCGGCCCGGCC
GCGGGGAGCCCCGCTTCATTGCAGTGGGCTACGTGGACGA
CACCCAGTTCGTGAGGTTCGACAGCGACGCCGCGAGTCCG
AGGACGGAGCCCCGGGCGCCATGGATAGAGCAGGAGGGGC
CGGAGTATTGGGACCGGAACACACAGATCTTCAAGACCAA
CACACAGACTTACCGAGAGAACCTGCGGATCGCGCTCCGC
TACTACAACCAGAGCGAGGCCGGGTCTCACACTTGGCAGA
CGATGTATGGCTGCGACGTGGGGCCGGACGGGCGCCTCCT
CCGCGGGCATAACCAGTACGCCTACGACGGCAAAGATTAC
ATCGCCCTGAACGAGGACCTGAGCTCCTGGACCGCGGCGG
```

```
ACACCGCGGCTCAGATCACCCAGCGCAAGTGGGAGGCGGC
CCGTGAGGCGGAGCAGCTGAGGGCCTACCTGGAGGGCCTG
TGCGTGGAGTGGCTCCGCAGACACCTGGAGAACGGGAAGG
AGACGCTGCAGGCCGCGGACCCCCCAAAGACACAGCTGAC
CCACCACCCCGTCTCTGACCATGAGGCCACCCTGAGGTGC
TGGGCCCTGGGCTTCTACCCTGCGGAGATCACACTGACCT
GGCAGCGGGATGGCGAGGACCAAACTCAGGACACTGAGCT
TGTGGAGACCAGACCAGCAGGAGATAGAACCTTCCAGAAG
TGGGCAGCTGTGGTGGTGCCTTCTGGAGAAGAGCAGAGAT
ACACATGCCATGTACAGCATGAGGGGCTGCCGAAGCCCCT
CACCCTGAGATGGGAGCCATCTTCCCAGTCCACCATCCCC
ATCGTGGGCATTGTTGCTGGCCTGGCTGTCCTAGCAGTTG
TGGTCATCGGAGCTGTGGTCGCTACTGTGATGTGTAGGAG
CAAGAGCTCAGTGGAAAAGGAGGGAGCTACTCTCAGGCTG
CGCCAGCGACAGTGCCCAGGGCTCTGATGTGTCTCTCACA
GCTTGA
```

This DNA codes for HLA-B51 antigens.

In base sequences above-said, and those of accompanied claims, and detailed expression of the invention, A is adenine, C is cytosine, G is guanine and T is thymine.

Another object of the present invention is to produce HLA class I DNA having exon of the following base sequence;

```
ATGCGGGTCACGGCGCCCCGAACCGTCCTCCTGCTGCTCT
GGGGGGCAGTGGCCCTGACCGAGACCTGGGCCGCTCCCAC
TCCATGAGGTATTTCTACACCGCCATGTCCCGGCCCGGCC
GCGGGGAGCCCCGCTTCATCGCAGTGGGCTACGTGGACGA
CACCCAGTTCGTGAGGTTCGACAGCGACGCCGCGAGTCCG
AGGACGGAGCCCCGGGCGCCATGGATAGAGCAGGAGGGGC
CGGAGTATTGGGACCGGGAGACACAGATCTCCAAGACCAA
CACACAGACTTACCGAGAGAACCTGCGGATCGCGCTCCGC
TACTACAACCAGAGCGAGGCCGGGTCTCACACTTGGCAGA
CGATGTATGGCTGCGACGTGGGGCCGGACGGGCGCCTCCT
CCGCGGGCATAACCAGTACGCCTACGACGGCAAAGATTAC
ATCGCCCTGAACGAGGACCTGAGCTCCTGGACCGCGGCGG
ACACCGCGGCTCAGATCACCCAGCGCAAGTGGGAGGCGGC
```

```
CCGTGAGGCGGAGCAGCTGAGGGCCTACCTGGAGGGCCTG
TGCGTGGAGTGGCTCCGCAGACACCTGGAGAACGGGAAGG
AGACGCTGCAGGCCGCGGACCCCCCAAAGACACACGTGAC
CCACCACCCCGTCTCTGACCATGAGGCCACCCTGAGGTGC
TGGGCCCTGGGCTTCTACCCTGCGGAGATCACACTGACCT
GGCAGCGGGATGGCGAGGACCAAACTCAGGACACTGAGCT
TGTGGAGACCAGACCAGCAGGAGATAGAACCTTCCAGAAG
TGGGCAGCTGTGGTGGTGCCTTCTGGAGAAGAGCAGAGAT
ACACATGCCATGTACAGCATGAGGGGCTGCCGAAGCCCCT
CACCCTGAGATGGGAGCCATCTTCCCAGTCCACCATCCCC
ATCGTGGGCATTGTTGCTGGCCTGGCTGTCCTAGCAGTTG
TGGTCATCGGAGCTGTGGTCGCTACTGTGATGTGTAGGAG
GAAGAGCTCAGTGGAAAAGGAGGGAGCTACTCTCAGGCTG
CGCCAGCGACAGTGCCCAGGGCTCTGATGTGTCTCTCACA
GCTTGA
```

This DNA codes for HLA-Bw52 antigens.

Both of HLA class I DNAs can be applied as DNA probes which are utilized for DNA typing by labelling a marker substance to a part of the DNAs.

Also, by introducing these DNAs into eucaryotic cells, transformed cells expressing HLA class I allotype can be obtained.

The second object of the present invention is to produce DNA probes comprising a part of either of said two HLA class I DNAs and a marker substance combined to said DNA.

These DNA probes are useful to study, examine, and diagnose HLA class I allotypes.

The third object of the invention is to produce transformed cells which express either HLA-B51 antigen or HLA-Bw52 antigen by introducing either of said two of HLA class I DNAs into eucaryoctic cells other than the human being.

Cells expressing allotype of HLA class I antigen can be administered to animals as immunogen and are therefore useful for producing monoclonal antibodies which show specificity to allotype of HLA class I antigen. Moreover, cells which are obtained by introducing the present DNA into eucaryoctic cells of animals other than human beings and express allotype of HLA class I antigen are useful as panel cells utilized for specificity judgement of antiserum.

This invention can be more fully understood from the following detailed description when taken in conjunction with the accompanying drawings, in which:

Fig. 1 shows exon-intron organization of HLA-B locus on the sixth human chromosome;

Figs. 2A-2G show complete sequences of HLA-B51 and HLA-Bw52;

Figs. 3A-3F show amino acid sequences which are coded by HLA-B51 and HLA-Bw52, respectively; and

Fig. 4 shows HLA gene region on the sixth human chromosome.

Examples of marker substances utilized for DNA probes of the invention include luminous materials such as peroxidase, iron-porphyrin derivatives, and luciferase, fluorescent materials, phosphorescent materials such as 2,3-butanedion, and radioactive materials such as $^{32}$P and $^{35}$S. These marker substances and a method for labelling these substances to a single-stranded DNA is known (Annual Review biophys. Bioeng. pp 175-195, (1981). Rigby et al., J. Mol. Biol., 113, 237, (1977)).

An example method for introducing HLA class I DNA of the invention into eucaryoctic cells is a usual method in the art, such as calcium phosphate technique.

Description of the Preferred Embodiments is as follows.

Isolation of HLA class I genes

separation of chromosome DNA

Five × 10$^7$ of human B cell strains LKT-2 (received from Dr. Ohtani at Kitazato Univ.) which include haplotypes of HLA-A2 2, B51 51, and C-·· and are transformed with EB virus (Epstein-Barr Virus) were suspended in a 10 mℓ of phosphate-buffered saline (PBS) and centrifuged at 1500 rpm. Next, a supernatant solution was removed, and the LKT-2 cells were further suspended in 10 mℓ of PBS, and centrifuged at 1500 rpm again, and the supernatant solution was then completely removed.

Next, LKT-2 cells were suspended in 2 mℓ of LST buffer solution (containing 20 mM of Tris solution at pH 7.4, 10 mM of NaCℓ, and 3 mM of MgCℓ$_2$), and centrifuged at 1500 rpm, followed by exclusion of a supernatant solution. To the resulting precipitate, 1 mℓ of LST buffer solution containing 5% Soccse and 4% NP-40, which is cooled at 4°C, was added to suspend LKT-2 cells. The obtained suspension was allowed to stand for 5 minutes. Next, the suspension was centrifuged at 1500 rpm to a supernatant solution, and 5 mℓ of cooled ACE buffer solution (50 mM sodium acetate and 10 mM of EDTA) was added with stirring. To the resultant suspension, 0.5 mℓ of 10% SDS solution and sequentially 5 mℓ of phenol solution were added and the resultant suspension was shaken for 4 minutes. After that, this suspension was centrifuged at 2000 rpm for 10 minutes and an upper layer was poured in a new test tube. To this tube, chloroform of an amount equivalent to the upper layer was added. The resultant mixture was shaken for four minutes and centrifuged at 2000 rpm for ten minutes. The obtained upper layer was poured into a new tube. To this tube, two-fold 99% ethanol was slowly added to separate thread-like human chromosome DNAs. Thirty micrograms of the separated human chromosome DNA was incubated at 37°C for three hours with 90 units (U) of restriction enzyme ECorl, thereby conducting DNA fragmentation. The resultant DNA fregments were deposited to the end region of 0.8% of agarose gel for electrophoresis to separate DNA fregment of 6.0 kd to 8.5 kd through DE paper separation. HLA class I genome DNA was thus obtained.

Said operations were performed to human normal peripheral blood lymphocyte (PBL) having haplotypes of HLA-All/24, Bw52/-, and Cw7/-. Consequently, HLA class I genome DNA was obtained from PBL cells.

Formation of Genome DNA Library

To 0.4μg of human DNA separated, 2 μg of arms of ramda phage (ramda ONGC treated with EcoRI, manufactured by Stratagene Co.) and T₄ ligase were added. The resultant mixture was incubated at 4°C for 18 hours for combining. This sample was packaged in vitro by utilizing a packaging kit (Gigapack-gold: mamnufactured by Stratagene Co.). In order to confirm that packaging was successfully performed, titer of packaged phage samples was measured by the following steps.

Measurement of Titre of the Sample

A small amount of LE 392 E.Coli was inoculated to 40 mℓ of LB medium (5 g of NaCℓ, 5 g of yeast extract, and 10 g of trypton were dissolved in 1ℓ H₂O) in a 50 mℓ centrifuge tube, and,the mixture was cultured with shaking at 37°C overnight. One mℓ of cultured bacteria medium was added in a centrifuge tube, and further 3 mℓ of LB medium and 80 mℓ of 1M MgSO₄ were added. 0.2 mℓ of the bacteria medium thus obtained and 2μℓ of respective diluted packaging sample (primitive concentrate, 10-fold diluted solution, 100-fold diluted solution, 1000-fold diluted solution) were added in each 6 mℓ test tubes, and each mixture was incubated at 37°C for 15 minutes. Into each test tube, 2.5 mℓ of 0.7% soft agar maintained at 45°C was added and mixed. The resultant mixture was layered on a 1.5% agar plate of 90 mm length. and cultured at 37°C for 8 hours. After culturing, the number of plaques was counted.

Cloning of HLA Class I Genome DNA

The primitive concentrate including the DNA library was diluted with SM medium (15.8 g of NaCℓ, 2 g of MgSO₄7H₂O, 50 mℓ of 1M TrisCℓ pH 7.5, and 5.5 mℓ of 2% gelatin were dissolved in 1ℓ of H₂O) so that 4000 plaques may be formed on the plate of 90 mm diameter. Primary screening was conducted by the same steps as in said titre measurement to form phage plaques on the plate of 90 mm diameter. A nitrocellulose membrane (manufactured by Gelmen Science Co.) was mounted on the soft agar layer and allowed to stand for five minutes. Next, the nitrocellulose membrane was placed on chromatography paper containing a denature solution (0.1M NaOH and 1.5M NaCℓ) such that a phages transferred-surface of the membrane was upwards, and allowed to stand for five minutes. The nitrocellulose membrane was then mounted on chromatography paper containing two-fold SSC solution, allowed to stand for five minutes, and dried at room temperature. Finally, the membrane was kept in a vacuum dryer at 80°C for 120 minutes to complete blotting.

HLA-B7cDNA probes for hybridization were prepared as follows.

Plasmid pDp001 (obtained from Dr. Weisman, at Yale University) which is pBR 328 including HLA-B7cDNA 1340bp (Orr H.T. et al; Biochemistry, 18, 5711, 1979) at PstI site was cleaved by PstI and electrophoresed on agarose gel to separate 1340bp of HLA-B7cDNA by DE paper technique. Two hundred nanograms of HLA-B7cDNA separated was labelled with ³²P-dCTP by nick translation. The labelled DNA was applied to Sephadex G50 column to separate labelled cDNA from noncombined ³²P-dCTP, and the first radioactive peak was collected.

HLA class I gene was cloned by the following steps.

(i) primary screening

The nitrocellulose membrane which blotted library-forming phages was placed in a hybridization bag. Prehybridization solution (0.8 mℓ of 50-fold Denhardt's solution, 10 mℓ of formamide, 0.2 mℓ of 10% SDS, 0.5 mℓ of 4 mg/mℓ salmon sperm DNA, 6.6 mℓ of 20-fold SSC, and 1.9 mℓ of H₂O) was poured in this bag. The bag was then sealed and incubated at 42°C for two hours. Two × 10¹⁷ cpm of HLA-B7cDNA probes and 0.5 mℓ of 4 mg/mℓ salmon sperm DNA were put into a glass test tube, and the tube was heated in a water bath at 100°C for 10 minutes and rapidly quenched in ices. To this glass tube, a mixed solution containing 0.8 mℓ of 50-fold Denhardt's solution, 10 mℓ of formamide, 0.2 mℓ of 10% SDS, 6.6 mℓ of 20-fold SSC solution, and 2 mℓ of 50% Dextran sulfate was added to prepare hybridization solution. Next, the prehybridization solution was removed from the hybridization bag, and the hybridization solution was added instead. The bag was then sealed and incubated at 42°C for 18 hours. Next, the nitrocellulose membrane was put out from the bag, washed with a washing solutioncontaining 2-fold SSC and 0.1% SDS at 37°C for 30 × 2 minutes, further washed at 52°C for 30 × 2 minutes and dried. This dried membrane was fixed on Whatman 3MM filter paper. The paper was mounted in a cassette of X-ray film, and the X-ray film was exposed to radiation emitted from labelled DNA probes for 3 - 18 hours to sensitize the film. After confirming positive plaques which appeared as black dots, these plaques were taken from the plate of 90 mm diameter with a pipett, put into a centrifuge tube of 1.5 m$\ell$ containing 1 m$\ell$ of SM buffer and incubated at 37° C for one hour. Next, to this tube, several droplets of chloroform were added and the tube was then centrifuged at 15000 rpm. The supernatant solution was taken as phages for secondary screening.

(ii) secondary screening

The supernatant solution obtained in (i) was diluted with SM medium such that 100-500 plaques were formed in the plate of 90 mm diameter. Next, these plaques were blotted on a nitrocellulose membrane by the same method as in primary screening, and hybridized with HLA-B7cDNA probes to confirm positive plaques. These positive plaques were taken with a pitett, put into a 1.5 m$\ell$-centrifuge tube containing 1 m$\ell$ of SM medium and incubated at room temperature for one hour. To this tube, several droplets of chloroform were poured and the tube was then centrifuged at 15000 rpm for several seconds. After centrifugation, the supernatant solution was taken as phages for third screening.

(iii) third screening

The supernatant solution obtained in (ii) was screened by the same method as in the secondary screening and hybridized to confirm that all of the plaques formed were positive plaques.

Purification of DNA

A titre of Rammda-phage obtained in the third screening were determined by the same steps as in said measuremenmt of the sample's titre. In order to increase the titre of phages obtained in third screening, phage plaques first were put in the 90 m$\ell$-plate. Second, 4 m$\ell$ of SM medium was added in the plate next day to incubate the plate for one hour. Next, the supernatant solution of the incubated medium was put into a 1.5 m$\ell$-centrifuge tube, and 50 $\mu\ell$ of chloroform was added into the tube. The resultant mixture was mixed for one hour and centrifuged at 15000 rpm for one minute. Phages in a supernatant solution of the centrifuged solution was titred by hybridization in the same method as in screening. The titre was 1.0 × $10^9$.

LE392 E.coli was put in 30 m$\ell$ of NZYDE medium containing 0.4% maltose, and the bacteria were cultured overnight. Next day, this medium was further centrifuged at 4000 rpm for five minutes, and the supernatant solution obtained was removed and 5 m$\ell$ of SM medium was added to suspend the bacteria. At this time, 4 × $10^{19}$ E.Coli were suspended in 5 m$\ell$ of the SM medium simultaneously with measurement of OD value at 600 nm utilizing a spectrophotometer (OD value: 1.0 = 8 × $10^8$ bacteria). Five milliliters of SM medium suspended with 4 × $10^{10}$ of E.coli and 1 × $10^9$ titred phages prepared above were put into a test tube and incubated at 37° C for two minutes. The incubated solution was added to 1$\ell$ of NZYDE medium and the resultant medium was cultured with shaking at 37° C for 8 hours. To the resulting medium, 10 m$\ell$ of chloroform was further added followed by shaking for 20 minutes. The medium obtained was put into a 1$\ell$ of centrifuge tube and centrifuged at 3500 rpm for 30 minutes with cooling. Next, the supernatant solution of the centrifuged medium was put into new tubes, and DNase and RNase were added into the tubes so as to obtain final concentrations of 1 $\mu$g/m$\ell$ respectively, followed by incubation at room temperature for 30 minutes. To this centrifuge tube, 36 g of NaC$\ell$ and 64 g of PEG 6000 (manufactured by Sigma Co.) were added and dissolved, and the tube was allowed to stand at 4° C for overnight. The tube was then centrifuged at 3500 rpm for 30 minutes and a supernatant solution of centrifuged solution was removed. To this tube, 6 m$\ell$ of SM medium was added, and the resultant mixture was transferred to another 50 m$\ell$-centrifuge tube, and added with 6 m$\ell$ of chloroform for mixing. After this 50 m$\ell$-tube was centrifuged at 4000 rpm, the resultant supernatant solution was transferred to another centrifuge tube. To this supernatant solution, SM medium was added to prepare 6 m$\ell$ of the entire solution, and 3 g of CsC$\ell$ was further added and well dissolved. The tube was centrifuged at 22000 rpm for four hours and phage layers were separated by CsC$\ell$ specific gravity centrifuge separation technique. Phage layer separated was added in SM medium containing 1.5 g/m$\ell$ CsC$\ell$ and the resultant medium was further centrifuged at 45000 rpm for 18 hours to separate phage layers. These phage layers in solution were dialyzed for one hour trough a dialysis membrane by utilizing a solution containing 0.1M Tris, 0.05M NaC$\ell$, and 0.001 M MgC$\ell_2$.

To dialyzed phage solution, 1/10 amounts of 0.5M EDTA, 1/20 amounts of 10% SDS, and 1/20 amounts of 2 mg/mℓ of Proteinase K were added and the resultant mixture was incubated at room temperature for 30 minutes. Next, DNA was separated by Phenol method and purified by dialysis overnight utilizing TE buffer solution (10 mM Tris Cℓ, and 1 mM EDTA pH 8.0).

Expression of HLA Class I Gene by Gene Introduction

HLA class I genome DNA cloned were introduced into tk- mouse L cells, and expression of the DNA on the cellular surface was detected by anti-HLA class I antibodies.

Introduction of HLA class I genes into mouse L cells

On the previous day of introgression, $5 \times 10^6$ mouse L cells were put into a 75 cm²-culture flask, and the cells were cultured in MEM medium containing 10% fetal calf serum (FCS).

Ca-phosphate precipitation method was conducted to form precipitates by the following steps.

(a) Twenty µg/100 µℓ of HLA class I genome DNA, 1 µg/100 µℓ of thymidine kinase gene, 100 µℓ of CaCℓ$_2 \cdot$2H$_2$O at pH 7.0, and 700 µℓ of H$_2$O were mixed to prepared 1 mℓ of solution.

(b) Hepes.NaCℓ solution and 1% NaHPO$_4$ were mixed at 49:1 ratio and the resultant mixture was put into a 6 mℓ-tube.

(c) The solution obtained in (a) was put into the tube in (b) drop by drop. The resultant mixture was incubated for 20 minutes and Ca-DNA precipitate was formed.

Mouse L cells cultured on the previous day were once washed with Ca-free PBS, and then added with Ca-DNA precipitate. The resultant mixture was incubated in a flask for 15 minutes. Ten mℓ of MEM medium containing 10% FCS was further added to the mixture, and the obtained mixture was incubated in a CO$_2$ incubator at 37°C for six hours. Next, after the medium in the flask was removed by 34% PEG-5% DMSO solution formed by using a FCS-free medium, 2 mℓ of 34% PEG-5% DMSO solution was added to the flask and the resultant suspension was incubated for three minutes. The obtained cells were washed with a FCS-free medium three times and further washed with a medium containing 10% FCS twice. Next, a medium containing 10 mℓ of 10% FCS was added and the cells were cultured overnight. On the next day, the present medium was replaced with HAT medium and the cells were cultured again. HAT medium was newly replaced two or three days apart. In approximately 14-21 days, tk gene-introduced L cells were grown and colony formation was confirmed. Each colony was taken with a cloning ring and each clone was cultured respectively.

Confirmation of HLA Class I Antigens Expressed on Transformed Cells

One × $10^6$ cloned cells of each colony were washed with PBS twice and suspended in 100 µℓ of PBS, and then 50 µℓ portions were put into two test tubes. Fifty µℓ of anti-HLA class I monoclonal antibodies W6/32 (Pharham P. et al., J. Immunol. (1979), 123, pp 342-349) and 50 µℓ of anti-HLA-DR monoclonal antibodies L 243 (Lampson L.A. et al., J. Immunol. (1980), 125, pp 293-299) were separately put into said test tubes, and incubated at 4°C for thirty minutes. To each tube, 0.5 mℓ of PBS was poured and the tube was centrifuged at 1500 rpm for 5 minutes and a supernatant solution of the centrifuged suspension was removed. This operation was repeated three times. Fifty-fold diluted solution of FITC anti-mouse Ig antibodies (manufactured by Silenus Co.) was added to each tube and incubated at 4°C for 30 minutes. To each tube, 0.5 mℓ of PBS was added and centrifuged at 1500 rpm for five minutes and then a supernatant solution of the centrifuged suspension was removed. This operation was also repeated three times. Finally, 1 mℓ of PBS was poured into each tube and the suspension was measured in fluorescence intensity utilizing a spectrum III (manufactured by Orsou Co.). L Cells on which HLA class I antigens were expressed could be confirmed, because fluorescent intensity of them was stronger with W6/32 antibodies than with L243 antibodies.

From aforementioned operations, it was confirmed that transformed cells introgressed with genome DNA derived from LKT-2 cells expressed two kinds of HLA class I genes: LKT-2-18 and LKT-2-21, and those introgressed with genome DNA derived form PBL cells expressed six kinds of HLA class I genes; A-2, A-11, L-1, L-2, L-3, and I-1.

Determination of Alloantigenicity of Cloned HLA Class I Genome DNA

It was suggested that HLA-2-18 separated from LKT-2 cells and L-2 separated from PBL cells were HLA-B51 and HLA-Bw52 antigen genes, from the fact that HLA-2-18 and L-2 have prominently similar restriction enzyme maps. In order to confirm this suggestion, mouse L cells introgressed with these genes were utilized in the absorption test of human anti-B51 serum and anti-B5 serum to determine alloantigenicity of them.

Five × 10⁵ of mouse L cells, mouse L cells introgressed with LKT-2-18 and mouse L cells introgressed with L-2 were separated put into 1.5 ml-centrifuge tubes, and centrifuged at 15000 rpm for one minute and a resultant supernatant solution was removed. By repeating this process, cells of each type were prepared in a plurality of tubes. To two centrifuge tubes including mouse L cells introgressed with LKT-2-18 (LKT-2-18/mouse L cells), anti-B5 antibodies and anti-B51 antibodies were added respectively. Also, to three centrifuge tubes including mouse L cells introgressed with L-2 (L-2/mouse cells) and other three centrifuge tubes including mouse L cells, anti-All serum, Anti-A24 serum and anti-B5 serum were added respectively. These tubes were incubated at 37°C for thirty minutes, followed by further incubation at 4°C for thirty minutes. Each tube was centrifuged at 15000 rpm for five minutes and the resultant supernatant solution was subjected to complement-dependent cytotoxic test. The results are shown in Table 1.

Table 1

| results of complement-dependent cytotoxic test | | | | | |
|---|---|---|---|---|---|
| | cytotoxic ratio after serum absorption (%) | | | | |
| absorption cells | target cell | anti-A11 | anti-A24 | anti-B5 | anti-B51 |
| mouse L cell | PBL | 100 | 50 | 100 | NT |
| | LKT-2 | NT | NT | 100 | 100 |
| L-2/mouse cell | PBL | 80 | 50 | 10 | NT |
| LKT-2-18/mouse L cell | LKT-2 | NT | NT | < 10 | 30 |
| remarks) PBL cell includes HLA-A11 24, Bw52⁻-, and Cw7⁻-. NT; non-tested. | | | | | |

Each human serum of anti-All, anti-A24, anti-B5, and anti-B51 were obtained from Dr. Zyuji of Blood Transfusion Department of Tokyo University Hospital.

From the above results, it was confirmed that LKT-2-18 was HLA-51B, and L-2 was HLA-Bw52.

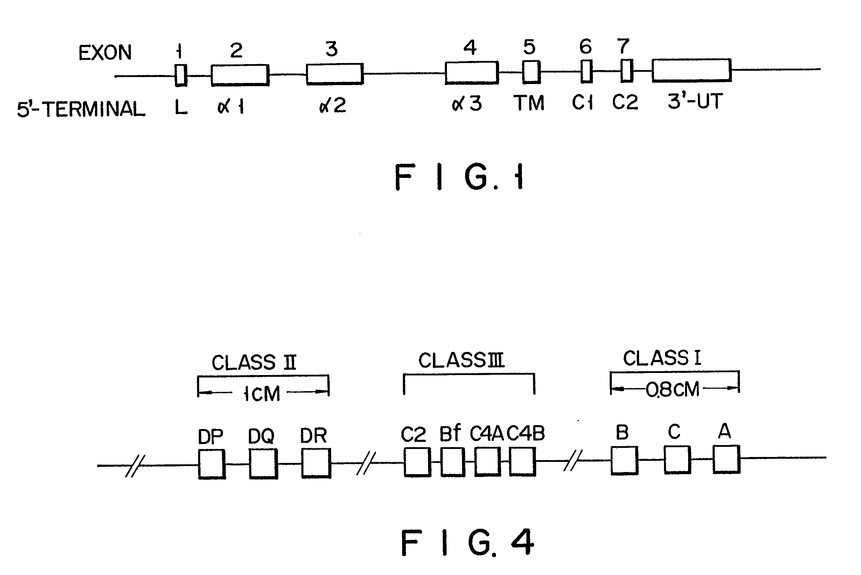

Fig. 1 shows exon-intron organization of HLA-B locus on human sixth chromosome. Here, exons 1-7 are assigned from 5'-terminal. exons 1-5 code for signal peptide, $\alpha_1$-domain, $\alpha_2$-domain, $\alpha_3$-domain, and TM region in turn, and exons 6 and 7 code for CY region.

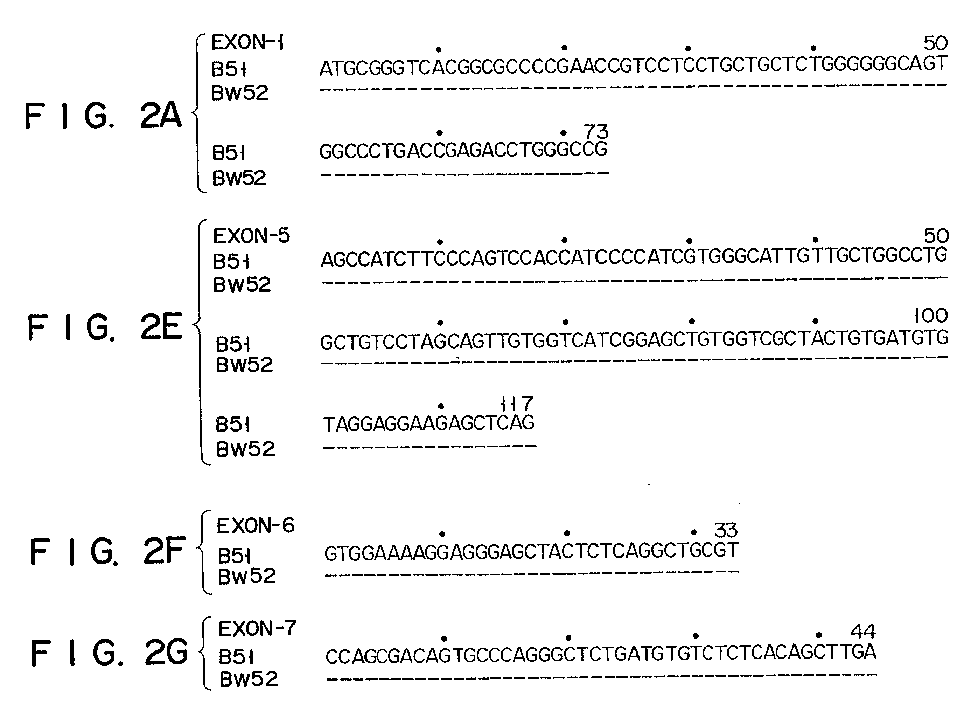

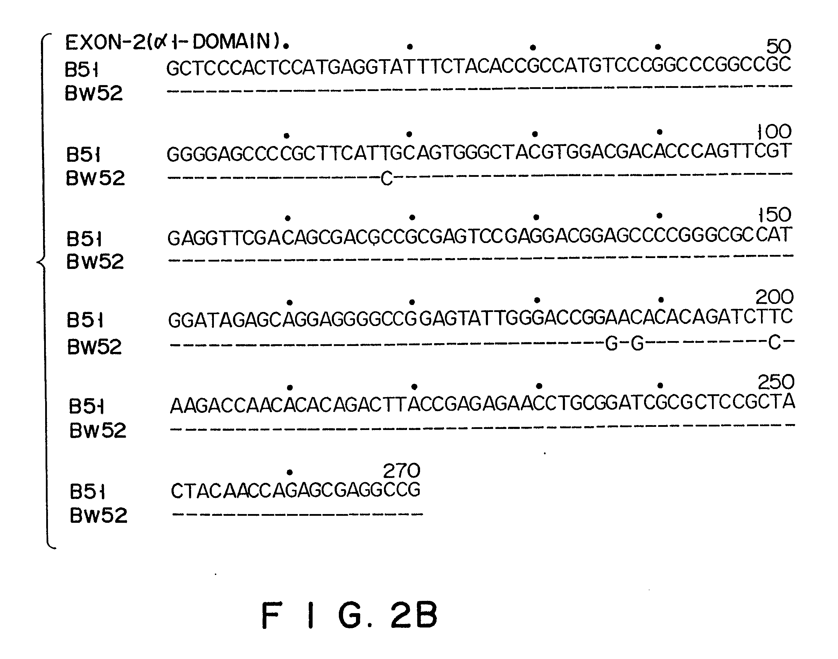

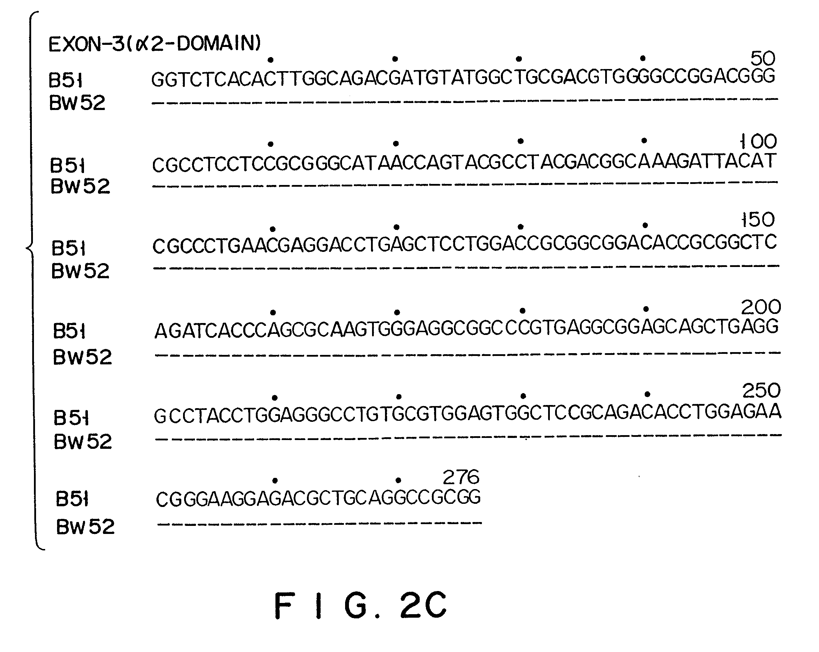

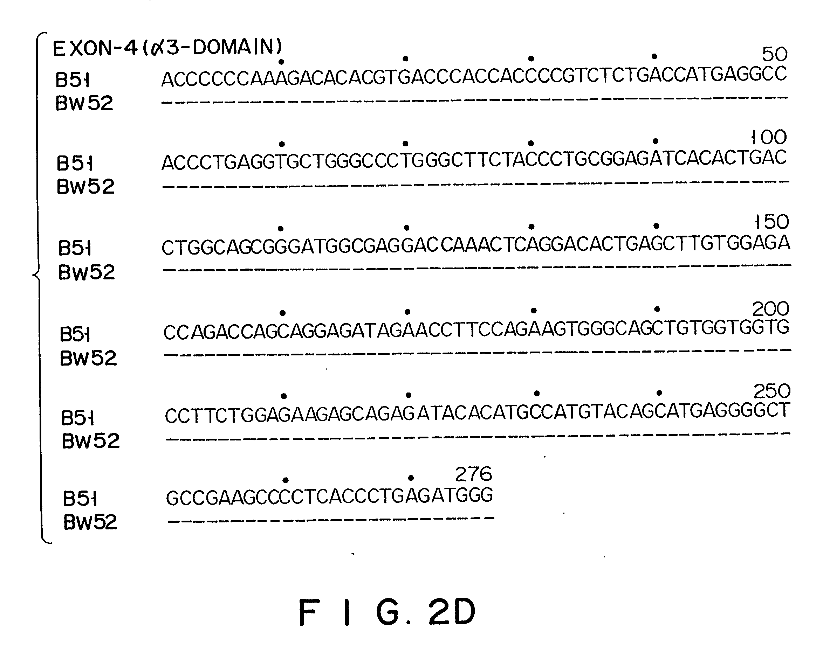

Total base sequences of genome DNA of LKT-2-18 and L-2 were determined by a deoxy method. The results are shown in Figs. 2A-2G. These sequences shown in Figs. 2A-2G correspond to those of exons 1-7. In these figures, LKT-2-18 is described as HLA-B51, and L-2 as HLA-Bw52. Further, in these figures, dashes in the HLA-Bw52 indicate the same sequence as HLA-B51.

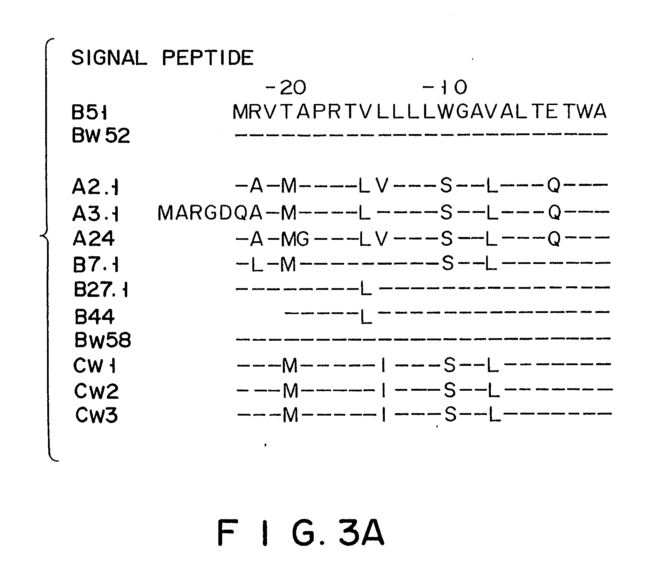

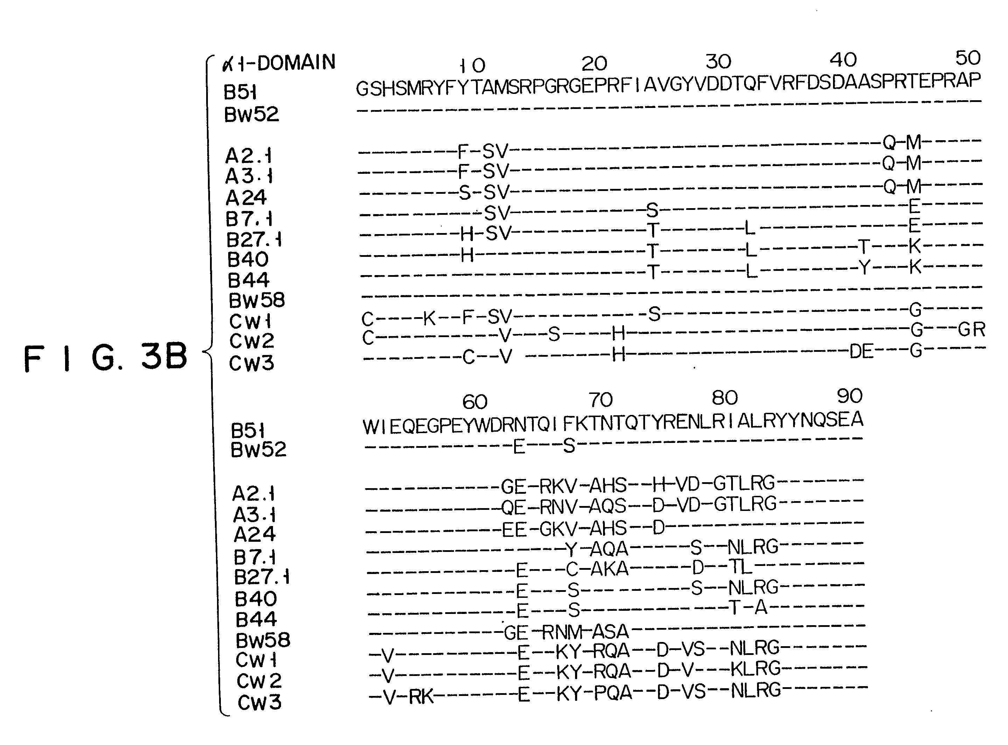

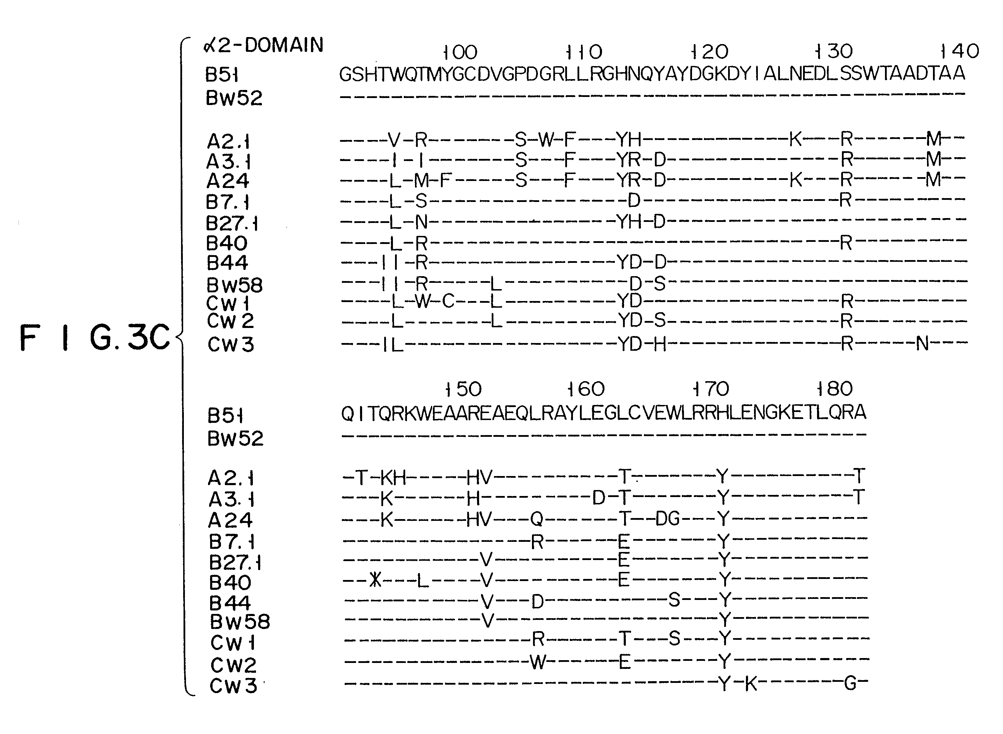

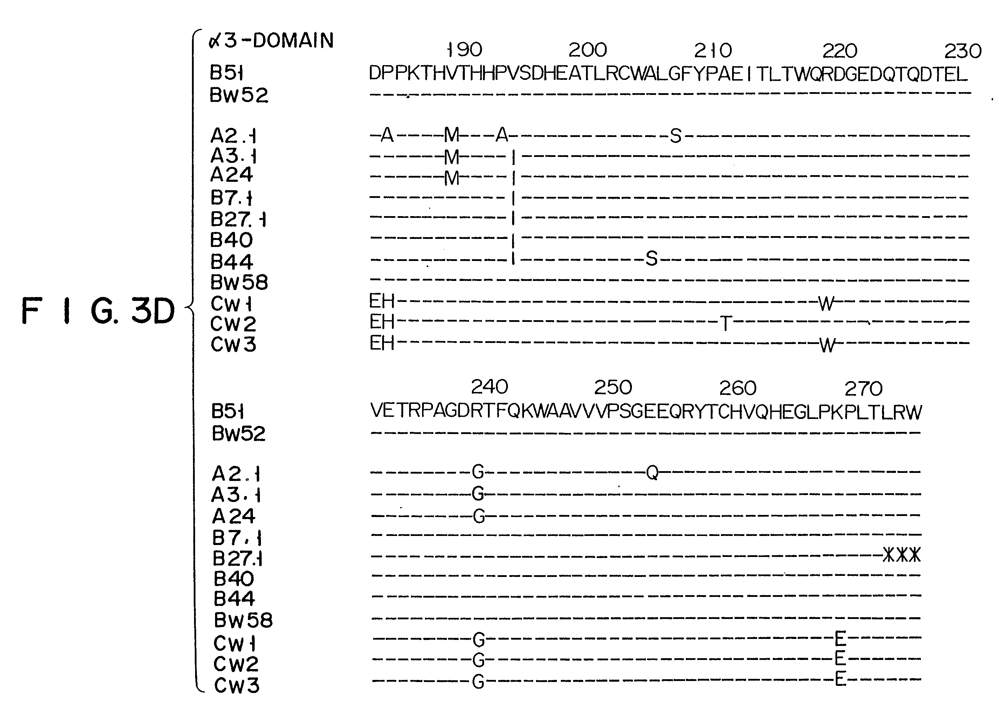

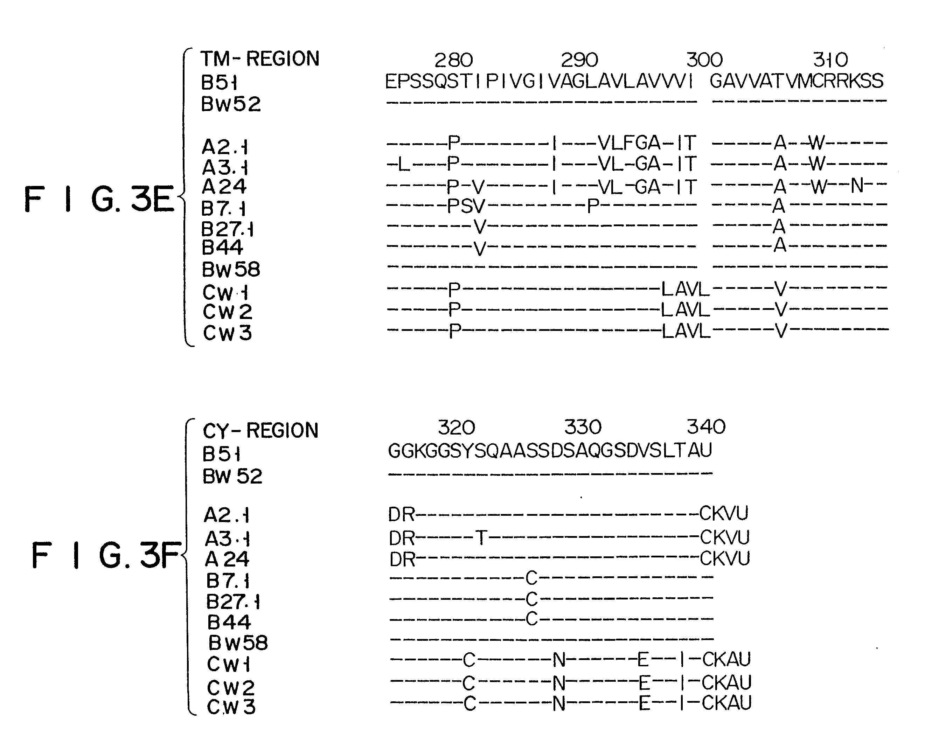

Figs. 3A-3F show amino acid sequences of peptides coded by exons of B51 and Bw52 shown in Figs. 2A-2G; Fig. 3A shows signal peptides, Fig. 3B shows amino acid sequences of $\alpha_1$-domain, Fig. 3C shows amino acid sequences of $\alpha_2$-domain, Fig. 3D shows amino acid sequences of $\alpha_3$-domain, Fig. 3E shows amino acid sequences of TM region, and Fig. 3F shows amino acid sequences of CY region. Each amino acid is shown as one-character abbreviation; A is alanine, R is arginine, N is asparagine, D is aspartic acid, C is cysteine, Q is glutamine, E is glutamic acid, G is glycine, H is histidine, I is isoleucine, L is leucine, K is lysine, M is methionine, F is phenylalanine, P is proline, S is serine, T is threonine, W is tryptophan, Y is tyrosine, and V is valine.

In these figures, amino acid sequences of allotype peptides of A2.1, A3.1, A24, A7.1, B27.1, B40, B44, and Bw58 are shown for reference.

A2.1 is known by Koller, B.H. et al: J. Immunol. (1985), 134, p 2727, A3.1 is known by Strachan, T. et al: EMBO J. (1984), 3, pp 887-894, A24 is known by N'Guyen, C. et al: Immunogenetics (1985), 21, 479, B7.1 is known by Orr H.T. et al: Biochemistry (1979), 18, 5711, B27.1 is known by Wan, A.M. et al: J. Immunol., (1986), 137, 3671, B40 and B44 are known by Ways, J.P. et al: Immunogenetics, (1987), 25, 323,

EP 0 354 580 A2

and Bw58 is known by Ways, J.P. et al: J. Biol. chem., (1985), 260 11924.

Application to DNA Probes

From the above embodiments, the base sequences of HLA-B51 and Bw52 are apparently obtained. Therefore, determination of allotypes of B51 and Bw52 is highly expected.

HLA-B51 and Bw52 were confirmed to have replacements of four nucleotides at exon 2 for coding $\alpha_1$ domain. The region including nucleotides whose replacement was confirmed in exon 2 is estimated to be specific base sequences for allotypes.

DNA utilized as probes are parts of HLA-B511 and Bw52, and are synthesized by a DNA synthesizer or phosphotriester synthesizing method on the basis of the specified base sequences. To synthesized DNA, alkaline phosphatase [3,1,3,1], polynucleotide kinase [2,7,1,78] and $\alpha$-$^{32}$P dATP are added to label 32P at 5'-terminal of a single-chain DNA. DNA probes are thus obtained.

Claims

1. HLA class I DNA having exons represented by the following base sequence;

10

ATGCGGGTCACGGCGCCCCGAACCGTCCTCCTGCTGCTCT

GGGGGGCAGTGGCCCTGACCGAGACCTGGGCCGCTCCCAC

TCCATGAGGTATTTCTACACCGCCATGTCCCGGCCCGGCC

GCGGGGAGCCCCGCTTCATTGCAGTGGGCTACGTGGACGA

CACCCAGTTCGTGAGGTTCGACAGCGACGCCGCGAGTCCG

AGGACGGAGCCCCGGGCGCCATGGATAGAGCAGGAGGGGC

CGGAGTATTGGGACCGGAACACACAGATCTTCAAGACCAA

CACACAGACTTACCGAGAGAACCTGCGGATCGCGCTCCGC

TACTACAACCAGAGCGAGGCCGGGTCTCACACTTGGCAGA

CGATGTATGGCTGCGACGTGGGGCCGGACGGGCGCCTCCT

CCGCGGGCATAACCAGTACGCCTACGACGGCAAAGATTAC

ATCGCCCTGAACGAGGACCTGAGCTCCTGGACCGCGGCGG

ACACCGCGGCTCAGATCACCCAGCGCAAGTGGGAGGCGGC

CCGTGAGGCGGAGCAGCTGAGGGCCTACCTGGAGGGCCTG

TGCGTGGAGTGGCTCCGCAGACACCTGGAGAACGGGAAGG

AGACGCTGCAGGCCGCGGACCCCCCAAAGACACAGCTGAC

CCACCACCCCGTCTCTGACCATGAGGCCACCCTGAGGTGC

TGGGCCCTGGGCTTCTACCCTGCGGAGATCACACTGACCT

GGCAGCGGGATGGCGAGGACCAAACTCAGGACACTGAGCT

TGTGGAGACCAGACCAGCAGGAGATAGAACCTTCCAGAAG

TGGGCAGCTGTGGTGGTGCCTTCTGGAGAAGAGCAGAGAT

ACACATGCCATGTACAGCATGAGGGGCTGCCGAAGCCCCT

CACCCTGAGATGGGAGCCATCTTCCCAGTCCACCATCCCC

ATCGTGGGCATTGTTGCTGGCCTGGCTGTCCTAGCAGTTG

TGGTCATCGGAGCTGTGGTCGCTACTGTGATGTGTAGGAG

GAAGAGCTCAGTGGAAAAGGAGGGAGCTACTCTCAGGCTG

CGCCAGCGACAGTGCCCAGGGCTCTGATGTGTCTCTCACA

GCTTGA

characterized in that A is adenine, C is cytosine, G is guanine, and T is thymine.

2. HLA class I DNA having exons represented by the following base sequence;

ATGCGGGTCACGGCGCCCCGAACCGTCCTCCTGCTGCTCT

GGGGGGCAGTGGCCCTGACCGAGACCTGGGCCGCTCCCAC
TCCATGAGGTATTTCTACACCGCCATGTCCCGGCCCGGCC
GCGGGGAGCCCCGCTTCATCGCAGTGGGCTACGTGGACGA
CACCCAGTTCGTGAGGTTCGACAGCGACGCCGCGAGTCCG
AGGACGGAGCCCCGGGCGCCATGGATAGAGCAGGAGGGGC
CGGAGTATTGGGACCGGGAGACACAGATCTCCAAGACCAA
CACACAGACTTACCGAGAGAACCTGCGGATCGCGCTCCGC
TACTACAACCAGAGCGAGGCCGGGTCTCACACTTGGCAGA
CGATGTATGGCTGCGACGTGGGGCCGGACGGGCGCCTCCT
CCGCGGGCATAACCAGTACGCCTACGACGGCAAAGATTAC
ATCGCCCTGAACGAGGACCTGAGCTCCTGGACCGCGGCGG
ACACCGCGGCTCAGATCACCCAGCGCAAGTGGGAGGCGGC
CCGTGAGGCGGAGCAGCTGAGGGCCTACCTGGAGGGCCTG
TGCGTGGAGTGGCTCCGCAGACACCTGGAGAACGGGAAGG
AGACGCTGCAGGCCGCGGACCCCCCAAAGACACACGTGAC
CCACCACCCCGTCTCTGACCATGAGGCCACCCTGAGGTGC
TGGGCCCTGGGCTTCTACCCTGCGGAGATCACACTGACCT
GGCAGCGGGATGGCGAGGACCAAACTCAGGACACTGAGCT
TGTGGAGACCAGACCAGCAGGAGATAGAACCTTCCAGAAG
TGGGCAGCTGTGGTGGTGCCTTCTGGAGAAGAGCAGAGAT
ACACATGCCATGTACAGCATGAGGGGCTGCCGAAGCCCCT
CACCCTGAGATGGGAGCCATCTTCCCAGTCCACCATCCCC
ATCGTGGGCATTGTTGCTGGCCTGGCTGTCCTAGCAGTTG
TGGTCATCGGAGCTGTGGTCGCTACTGTGATGTGTAGGAG
GAAGAGCTCAGTGGAAAAGGAGGGAGCTACTCTCAGGCTG
CGCCAGCGACAGTGCCCAGGGCTCTGATGTGTCTCTCACA
GCTTGA

characterized in that A is adenine, C is cytosine, G is guanine, and T is thymine.

3. HLA class I DNA which codes for the following amino acid sequences;

MRVTAPRTVLLLLWGAVALTETWA
GSHSMRYFYTAMSRPGRGEPRFIAVGYVDDTQFVRFDSDA
ASPRTEPRAPWIEQEGPEYWDRNTQIFKTNTQTYRENLRI
ALRYYNQSEAGSHTWQTMYGCDVGPDGRLLRGHNQYAYDG
KDYIALNEDLSSWTAADTAAQITQRKWEAAREAEQLRAYL
EGLCVEWLRRHLENGKETLQRADPPKTHVTHHPVSDHEAT

```
LRCWALGFYPAEITLTWQRDGEDQTQDTELVETRPAGDRT
FQKWAAVVVPSGEEQRYTCHVQHEGLPKPLTLRWEPSSQS
TIPIVGIVAGLAVLAVVVIGAVVATVMCRRKSSGGKGGSY
SQAASSDSAQGSDVSLTA
```

characterized in that in the above sequence, A is alanine, R is arginine, N is asparagine, D is aspartic acid, C is cystein, Q is glutamine, E is glutamic acid, G is glycine, H is hystidine, I is isoleucin, L is leucin, K is lysine, M is methionine, F is phenylalanine, P is proline, S is serine, T is threonine, W is tryptophan, Y is tyrosine, and V is valine.

4. HLA class I DNA which codes for the following amino acid sequences;

```
MRVTAPRTVLLLLWGAVALTETWA
GSHSMRYFYTAMSRPGRGEPRFIAVGYVDDTQFVRFDSDA
ASPRTEPRAPWIEQEGPEYWDRETQISKTNTQTYRENLRI
ALRYYNQSEAGSHTWQTMYGCDVGPDGRLLRGHNQYAYDG
KDYIALNEDLSSWTAADTAAQITQRKWEAAREAEQLRAYL
EGLCVEWLRRHLENGKETLQRADPPKTHVTHHPVSDHEAT
LRCWALGFYPAEITLTWQRDGEDQTQDTELVETRPAGDRT
FQKWAAVVVPSGEEQRYTCHVQHEGLPKPLTLRWEPSSQS
TIPIVGIVAGLAVLAVVVIGAVVATVMCRRKSSGGKGGSY
SQAASSDSAQGSDVSLTA
```

characterized in that A is alanine, R is arginine, N is asparagine, D is aspartic acid, C is cysteine, Q is glutamine, E is glutamic acid, G is glycine, H is hystidine, I is isoleucin, L is leucin, K is lysine, M is methionine, F is phenylalanine, P is proline, S is serine, T is threonine, W is tryptophane, Y is tyrosine, and V is valine.

5. DNA probe comprising a part of HLA class I DNA according to claim 1 and a marker substance combined to the part of the DNA.

6. DNA probe comprising a part of HLA class I DNA according to claim 2 and a marker substance combined to the part of the DNA.

7. Transformed cells which are obtained by introducing HLA class I DNA according to claim 1 into eucaryotic cells of animals other than human beings, and express HLA-B51 antigens.

8. Transformed cells which are obtained by introducing HLA class I DNA according to claim 2 into eucaryotic cells of animals other than human beings, and express HLA-Bw52 antigens.

EXON    1    2     3      4   5   6   7

5'-TERMINAL    L    $\alpha$1     $\alpha$2      $\alpha$3   TM   C1   C2   3'-UT

## F I G. 1

CLASS II         CLASS III         CLASS I

|← 1CM →|                 |← 0.8CM →|

DP   DQ   DR     C2   Bf   C4A   C4B     B   C   A

## F I G. 4

EP 0 354 580 A2

FIG. 2A
{
EXON-1
B51  ATGCGGGTCACGGCGCCCCGAACCGTCCTCCTGCTGCTCTGGGGGGCAGT  50
Bw52  --------------------------------------------------

B51  GGCCCTGACCGAGACCTGGGCCG  73
Bw52  -----------------------
}

FIG. 2E
{
EXON-5
B51  AGCCATCTTCCCAGTCCACCATCCCCATCGTGGGCATTGTTGCTGGCCTG  50
Bw52  --------------------------------------------------

B51  GCTGTCCTAGCAGTTGTGGTCATCGGAGCTGTGGTCGCTACTGTGATGTG  100
Bw52  --------------------------------------------------

B51  TAGGAGGAAGAGCTCAG  117
Bw52  -----------------
}

FIG. 2F
{
EXON-6
B51  GTGGAAAAGGAGGGAGCTACTCTCAGGCTGCGT  33
Bw52  ---------------------------------
}

FIG. 2G
{
EXON-7
B51  CCAGCGACAGTGCCCAGGGCTCTGATGTGTCTCTCACAGCTTGA  44
Bw52  --------------------------------------------
}

EXON-2(α1-DOMAIN).                                                                    50
B51    GCTCCCACTCCATGAGGTATTTCTACACCGCCATGTCCCGGCCCGGCCGC
Bw52   ——————————————————————————————————————————————————

                                                                                   100
B51    GGGGAGCCCCGCTTCATTGCAGTGGGCTACGTGGACGACACCCAGTTCGT
Bw52   ——————————————————————C———————————————————————————

                                                                                   150
B51    GAGGTTCGACAGCGACGCCGCGAGTCCGAGGACGGAGCCCCGGGCGCCAT
Bw52   ——————————————————————————————————————————————————

                                                                                   200
B51    GGATAGAGCAGGAGGGGCCGGAGTATTGGGACCGGAACACACAGATCTTC
Bw52   ——————————————————————————————————————G—G—————————————C—

                                                                                   250
B51    AAGACCAACACACAGACTTACCGAGAGAACCTGCGGATCGCGCTCCGCTA
Bw52   ——————————————————————————————————————————————————

                            270
B51    CTACAACCAGAGCGAGGCCG
Bw52   ————————————————————

# F I G. 2B

EP 0 354 580 A2

EXON-3(α2-DOMAIN)

```
                                                                    50
B51   GGTCTCACACTTGGCAGACGATGTATGGCTGCGACGTGGGGCCGGACGGG
Bw52  ——————————————————————————————————————————————————

                                                                    100
B51   CGCCTCCTCCGCGGGCATAACCAGTACGCCTACGACGGCAAAGATTACAT
Bw52  ——————————————————————————————————————————————————

                                                                    150
B51   CGCCCTGAACGAGGACCTGAGCTCCTGGACCGCGGCGGACACCGCGGCTC
Bw52  ——————————————————————————————————————————————————

                                                                    200
B51   AGATCACCCAGCGCAAGTGGGAGGCGGCCCGTGAGGCGGAGCAGCTGAGG
Bw52  ——————————————————————————————————————————————————

                                                                    250
B51   GCCTACCTGGAGGGCCTGTGCGTGGAGTGGCTCCGCAGACACCTGGAGAA
Bw52  ——————————————————————————————————————————————————

                                      276
B51   CGGGAAGGAGACGCTGCAGGCCGCGG
Bw52  ——————————————————————————
```

# F I G. 2C

EP 0 354 580 A2

EXON-4 (α3-DOMAIN)

```
                                                                        50
B51    ACCCCCCAAAGACACACGTGACCCACCACCCCGTCTCTGACCATGAGGCC
Bw52   ──────────────────────────────────────────────────

                                                                       100
B51    ACCCTGAGGTGCTGGGCCCTGGGCTTCTACCCTGCGGAGATCACACTGAC
Bw52   ──────────────────────────────────────────────────

                                                                       150
B51    CTGGCAGCGGGATGGCGAGGACCAAACTCAGGACACTGAGCTTGTGGAGA
Bw52   ──────────────────────────────────────────────────

                                                                       200
B51    CCAGACCAGCAGGAGATAGAACCTTCCAGAAGTGGGCAGCTGTGGTGGTG
Bw52   ──────────────────────────────────────────────────

                                                                       250
B51    CCTTCTGGAGAAGAGCAGAGATACACATGCCATGTACAGCATGAGGGGCT
Bw52   ──────────────────────────────────────────────────

                                                       276
B51    GCCGAAGCCCCTCACCCTGAGATGGG
Bw52   ────────────────────────────
```

# F I G. 2D

EP 0 354 580 A2

SIGNAL PEPTIDE

```
                      -20               -10
B51          M R V T A P R T V L L L L W G A V A L T E T W A
Bw52         — — — — — — — — — — — — — — — — — — — — — — —

A2.1           — A — M — — — L V — — — S — — L — — — Q — — —
A3.1         M A R G D Q A — M — — — — L — — — — S — — L — — — Q — — —
A24            — A — M G — — — L V — — — S — — L — — — Q — — —
B7.1           — L — M — — — — — — — — — — S — — L — — — — — —
B27.1          — — — — — — — — L — — — — — — — — — — — — — —
B44                  — — — — — L — — — — — — — — — — — — — —
Bw58         — — — — — — — — — — — — — — — — — — — — — — — —
Cw1            — — M — — — — — — I — — — S — — L — — — — — — —
Cw2            — — M — — — — — I — — — S — — L — — — — — — —
Cw3            — — — M — — — — — — I — — — S — — L — — — — — — —
```

F I G. 3A

EP 0 354 580 A2

EP 0 354 580 A2

# FIG. 3B

α1-DOMAIN
```
                        10        20        30        40        50
B51       GSHSMRYFYTAMSRPGRGEPRFIAVGYVDDTQFVRFDSDAASPRTEPRAP
Bw52      -------------------------------------------------

A2.1      -----------F-SV--------------------------------Q-M-----
A3.1      -----------F-SV--------------------------------Q-M-----
A24       -----------S-SV--------------------------------Q-M-----
B7.1      ------------SV------------S----------------------E-----
B27.1     --------H-SV---------T--------L------------------E-----
B40       --------H----------------T--------L------------T---K-----
B44       -------------------------T--------L------------Y---K-----
Bw58      -------------------------------------------------------
Cw1       C----K--F-SV----------------S------------------G-----
Cw2       C----------V---S----H---------------------------G----GR
Cw3       --------C--V ------------H--------------------DE---G-----
```

```
                        60        70        80        90
B51       WIEQEGPEYWDRNTQIFKTNTQTYRENLRIALRYYNQSEA
Bw52      -----------------E---S-------------------

A2.1      -----------------GE-RKV-AHS--H-VD-GTLRG---------
A3.1      -----------------QE-RNV-AQS--D-VD-GTLRG---------
A24       -----------------EE-GKV-AHS--D-----------------
B7.1      ---------------Y-AQA-----S--NLRG-----------
B27.1     ---------------E---C-AKA-----D--TL----------
B40       ---------------E---S----------S--NLRG-------
B44       ---------------E---S-----------T-A----------
Bw58      -----------------GE-RNM-ASA-----------------
Cw1       -V---------------E--KY-RQA--D-VS--NLRG-------
Cw2       -V---------------E--KY-RQA--D-V---KLRG-------
Cw3       -V-RK------------E--KY-PQA--D-VS--NLRG-------
```

# F I G. 3C

EP 0 354 580 A2

```
α2-DOMAIN            100        110        120        130        140
B51         GSHTWQTMYGCDVGPDGRLLRGHNQYAYDGKDYIALNEDLSSWTAADTAA
Bw52        -------------------------------------------------

A2.1        ----V-R-------S-W-F---YH-----------K---R------M--
A3.1        ----I-I-------S---F---YR-D-------------R------M--
A24         ----L-M-F-----S---F---YR-D--------K---R-----M--
B7.1        ----L-S---------------D-----------------R--------
B27.1       ----L-N---------------YH-D----------------------
B40         ---L-R----------------------------R------------
B44         ---II-R---------------YD-D----------------------
Bw58        ---II-R----L----------D-S-----------------------
Cw1         ----L-W-C---L---------YD---------------R--------
Cw2         ----L-------L---------YD-S-------------R--------
Cw3         ---IL-----------------YD-H-------------R-----N---

                     150        160        170        180
B51         QITQRKWEAAREAEQLRAYLEGLCVEWLRRHLENGKETLQRA
Bw52        -----------------------------------------

A2.1        -T-KH-----HV------------T--------Y--------T
A3.1        --K-------H---------D-T--------Y--------T
A24         --K-----HV----Q-------T--DG---Y----------
B7.1        ----------------R-----E-------Y-----------
B27.1       ---------------V------E-------Y-----------
B40         --X---L----V---------E-------Y-----------
B44         ---------------V--D-----------S---Y-------
Bw58        ---------------V--------------Y-----------
Cw1         ---------------R-----T--S---Y-------------
Cw2         ---------------W-----E-------Y-----------
Cw3         --------------------------------Y-K----G-
```

F I G. 3D

EP 0 354 580 A2

```
                            190        200        210        220        230
α3-DOMAIN
B51          DPPKTHVTHHPVSDHEATLRCWALGFYPAEITLTWQRDGEDQTQDTEL
Bw52         ------------------------------------------------

A2.1         -A---M--A-------------S--------------------------
A3.1         -----M----I-------------------------------------
A24          -----M----I-------------------------------------
B7.1         ----------I-------------------------------------
B27.1        ----------I-------------------------------------
B40          ----------I-------------------------------------
B44          ----------I---------S----------------------------
Bw58         ------------------------------------------------
Cw1          EH------------------------------W---------------
Cw2          EH-----------------------T-----------------------
Cw3          EH------------------------------W---------------


                            240        250        260        270
B51          VETRPAGDRTFQKWAAVVVPSGEEQRYTCHVQHEGLPKPLTLRW
Bw52         -------------------------------------------

A2.1         ---------G---------------Q------------------
A3.1         ---------G---------------------------------
A24          ---------G---------------------------------
B7.1         -------------------------------------------
B27.1        ----------------------------------------XXX
B40          -------------------------------------------
B44          -------------------------------------------
Bw58         -------------------------------------------
Cw1          ---------G-----------------------------E----
Cw2          ---------G-----------------------------E----
Cw3          ---------G-----------------------------E----
```

EP 0 354 580 A2

**F I G. 3E**

| | 280 | 290 | 300 | 310 |
|---|---|---|---|---|
| TM- REGION | | | | |
| B51 | EPSSQSTIPIVGIVAGLAVLAVVVI | | GAVVATVMCRRKSS | |
| Bw52 | ————————————————————— | | ————————————— | |
| A2.1 | ————P————————I———VLFGA–IT | | ——————A––W————— | |
| A3.1 | –L———P————————I———VL–GA–IT | | ——————A––W————— | |
| A24 | ————P–V—————I———VL–GA–IT | | ——————A––W——N—— | |
| B7.1 | ————PSV——————————P———————— | | ——————A———————— | |
| B27.1 | ————————V————————————————— | | ——————A———————— | |
| B44 | ————————V————————————————— | | ——————A———————— | |
| Bw58 | ————————————————————————— | | ————————————— | |
| Cw1 | ————P————————————LAVL—————V————— | | | |
| Cw2 | ————P————————————LAVL—————V————— | | | |
| Cw3 | ————P————————————LAVL—————V————— | | | |

**F I G. 3F**

| | 320 | 330 | 340 |
|---|---|---|---|
| CY- REGION | | | |
| B51 | GGKGGSYSQAASSDSAQGSDVSLTAU | | |
| Bw52 | ————————————————————————— | | |
| A2.1 | DR————————————————————————CKVU | | |
| A3.1 | DR—————T——————————————————CKVU | | |
| A24 | DR————————————————————————CKVU | | |
| B7.1 | ————————————C——————————— | | |
| B27.1 | ————————————C——————————— | | |
| B44 | ————————————C——————————— | | |
| Bw58 | ————————————————————————— | | |
| Cw1 | ——————C——————N——————E——I–CKAU | | |
| Cw2 | ——————C——————N——————E——I–CKAU | | |
| Cw3 | ———————C——————N——————E——I–CKAU | | |